# EUROPEAN PATENT APPLICATION

(11) **EP 4 813 918 A1**
(43) Date of publication of application: **30.09.2026**
(21) Application number: 24894090.0
(22) Date of filing: 15.11.2024
(51) Int. Cl.: C01B 39/38, B01J 29/40, B01J 35/60, B01J 35/63, C07B 61/00, C07C 1/20, C07C 15/08

(54) **ZEOLITE**

(30) Priority: 21.11.2023 JP 2023197540
(71) Applicant: Tosoh Corporation, Yamaguchi-ken 746-8501 (JP)
(72) Inventor: CHEN, Ning, Shunan-shi, Yamaguchi 746-8501 (JP); JONOO, Yuki, Shunan-shi, Yamaguchi 746-8501 (JP); SHINODA, Miyuki, Shunan-shi, Yamaguchi 746-8501 (JP)
(74) Representative: TBK
(86) International application number: PCT/JP2024/040580
(87) International publication number: WO 2025/110099

(57) **Abstract**

An object is to provide at least one of a zeolite having an oxygen ten-membered ring structure with which p-xylene selectivity can be increased in the manufacture of xylene by alcohol reforming and that, at the same time, can be adapted to industrial processes at low cost, a catalyst for alcohol reforming containing it or a method for manufacturing xylene using it.

A zeolite having an oxygen ten-membered ring structure comprises one or more elements selected from the group of magnesium, calcium, titanium, boron and phosphorus. The total pore volume of the zeolite is 0.21 cm³/g or less.

## Description

### Technical Field

The present disclosure relates to a zeolite containing a specified element.

### Background Art

One method for manufacturing p-xylene, which is a source material for terephthalic acid, is a manufacturing method using methanol reforming in which a zeolite containing a specific metal and having an oxygen ten-membered ring structure is used as a catalyst.

For example, in NPL 1, it is disclosed that aromatic hydrocarbons including p-xylene can be obtained by methanol reforming using zinc-containing ZSM-5 (an MFI zeolite) as a catalyst.

In PTL 1, furthermore, there is disclosed a method for co-producing p?xylene and ethylene propylene from toluene and methanol using, as a catalyst, an MFI zeolite modified with silicon using an organic silicon precursor.

### Citation List

### Patent Literature

PTL 1: Japanese Unexamined Patent Application Publication (Translation of PCT Application) No. 2017-518995

### Non Patent Literature

NPL 1: Catalysis Today, Volume 233, 2014, Pages 8-13

### Summary of Invention

### Technical Problem

In alcohol reforming methods like those described in PTL 1 and NPL 1, not only p-xylene but also aromatic hydrocarbons other than p-xylene are manufactured simultaneously. Although it is easy to separate xylene from the aromatic hydrocarbons, the separated xylene is a mixture of xylene isomers, namely p-xylene, o-xylene and m-xylene. Separating and purifying the intended p-xylene alone from such a mixture of xylene isomers requires a large amount of energy. The manufacture of xylene by alcohol reforming, therefore, also faces a need for a high percentage of p-xylene relative to the manufactured xylene. In the method of NPL 1, however, the percentage of p-xylene relative to the manufactured xylene ((hereinafter also referred to as "p-xylene selectivity") has been low.

In PTL 1, on the other hand, p-xylene selectivity is high compared with that in NPL 1, but the silicon modification of the MFI zeolite requires the use of a large amount of an expensive organic silicon precursor. In addition to this, generation of waste fluid containing organic silicon is inevitable, necessitating equipment for detoxifying the waste fluid containing organic silicon. The manufacturing method of PTL 1, therefore, involves high manufacturing cost and has been difficult to apply to industrial processes.

An object of the present disclosure is to provide at least one of a zeolite having an oxygen ten-membered ring structure with which p-xylene selectivity can be increased in the manufacture of xylene by alcohol reforming and that, at the same time, can be adapted to industrial processes at low cost, a catalyst for alcohol reforming containing it or a method for manufacturing xylene using it.

### Solution to Problem

To improve p-xylene selectivity in the manufacture of xylene by alcohol reforming, the inventors focused on zeolite catalysts and investigated their structures and compositions. As a result, it was found that by allowing a zeolite having an oxygen ten-membered ring structure to contain a specified element and setting its total pore volume equal to or smaller than a specified value at the same time, the percentage of p-xylene relative to the manufactured xylene (p-xylene selectivity) can be increased.

The present invention, therefore, is as described in the claims, and the gist of the present disclosure is as follows.
[1] A zeolite having an oxygen ten-membered ring structure, the zeolite comprising one or more specified elements selected from the group of magnesium, calcium, titanium, boron and phosphorus, wherein a total pore volume is 0.21 cm³/g or less.
[2] The zeolite according to [1], wherein the zeolite having an oxygen ten-membered ring structure is an MFI zeolite, an MEL zeolite, a TON zeolite, an STF zeolite, an MTT zeolite, an MWW zeolite or a ZSM-48 zeolite.
[3] The zeolite according to [1] or [2], wherein a molar ratio of silica to alumina is 10 or greater and 250 or less.
[4] The zeolite according to any one of [1] to [3], further comprising one or more additional elements selected from the group of silver, zinc, gallium and iron.
[5] The zeolite according to any one of [1] to [4], wherein the specified elements are supported at least on an external surface.
[6] The zeolite according to any one of [1] to [5], wherein in a difference spectrum obtained by subtracting an IR spectrum of the zeolite before adsorption of 2,6-di-tert-butylpyridine from an IR spectrum of the zeolite with 2,6-di-tert-butylpyridine adsorbed thereonto, a ratio of a maximum intensity of a peak having a peak top within a range of 1630 cm⁻¹ to 1650 cm⁻¹, inclusive, to a maximum intensity of a peak having a peak top within a range of 1600 cm⁻¹ to 1620 cm⁻¹, inclusive, is greater than 1.5.
[7] The zeolite according to any one of [1] to [6], wherein when the zeolite is subjected to a treatment of bringing a gas mixture of nitrogen and methanol into contact therewith under conditions of a reaction temperature of 420°C, a reaction pressure (gauge pressure) of 0.2 MPa, a duration of reaction of 6 hours and a methanol weight hourly space velocity of 1.0 Hr⁻¹, p-xylene selectivity is 40% or more.
[8] A catalyst for alcohol reforming, the catalyst comprising the zeolite according to any one of [1] to [7].
[9] A method for manufacturing xylene, the method comprising bringing the zeolite according to any one of [1] to [7] and a fluid containing an alcohol into contact.
[10] A method for co-producing benzene, xylene and toluene, the method comprising bringing the zeolite according to any one of [1] to [7] and a fluid containing an alcohol into contact.
[11] Use of a zeolite having an oxygen ten-membered ring structure for manufacturing xylene from an alcohol, the zeolite containing one or more specified elements selected from the group of magnesium, calcium, titanium, boron and phosphorus and having a total pore volume of 0.21 cm³/g or less.
[12] Use of a zeolite having an oxygen ten-membered ring structure for co-producing benzene, xylene and toluene from an alcohol, the zeolite containing one or more specified elements selected from the group of magnesium, calcium, titanium, boron and phosphorus and having a total pore volume of 0.21 cm³/g or less.

### Advantageous Effects of Invention

According to the present disclosure, there can be provided at least one of a zeolite with which p-xylene selectivity can be increased in the manufacture of xylene by alcohol reforming, a catalyst for alcohol reforming containing it or a method for manufacturing xylene using it. Description of Embodiments

Terms in this embodiment are as defined below.

An "aluminosilicate" is a complex oxide having a structure composed of repeated networks of aluminum (Al) and silicon (Si) with oxygen (O) interposed therebetween. Of aluminosilicates, those having crystalline powder X-ray diffraction (Hereinafter also referred to as "XRD.") peaks in their XRD patterns are "crystalline aluminosilicates," and those having no crystalline XRD peak are "amorphous aluminosilicates."

In this embodiment, an example of an XRD pattern is one obtained from an XRD measurement under the following conditions.
Accelerating current and voltage: 10 mA and 30 kV
X-ray source: CuKα radiation (λ = 1.54178 Å)
Measurement mode: continuous scanning
Scan condition: 2°/min
Measurement range: 2θ = 10° to 70°
Scattering slit: 1/3°
Divergence slit: 1/3°
Receiving slit: 0.3 mm
Filter: a Ni filter

The XRD pattern can be measured using a commonly used powder X-ray diffractometer (e.g., Ultima IV Protectus, manufactured by Rigaku Corporation). The crystalline XRD peaks, furthermore, are peaks that are detected with identified peak-top 2θ in an analysis of the XRD pattern using commonly used analysis software (e.g., IGOR Pro 8, manufactured by WaveMetrics, Inc.), and examples include XRD peaks having a half-width of 2θ = 0.50° or less.

A "zeolite" is a compound having an ordered structure in which framework atoms (Hereinafter also referred to as "T atoms.") are arranged with oxygen (O) interposed therebetween, and is a compound in which the T atoms are at least one of metal atoms or metalloid atoms. The metal atoms can be, for example, atoms of one or more selected from the group consisting of aluminum (Al), titanium (Ti), iron (Fe), zinc (Zn), gallium (Ga) and tin (Sn), with aluminum being preferred. The metalloid atoms can be, for example, atoms of one or more selected from the group of boron (B), silicon (Si), germanium (Ge), arsenic (As), antimony (Sb) and tellurium (Te), with silicon being preferred. It should be noted that zeolites in which the T atoms are substantially atoms of aluminum (Al) and silicon (Si) fall under the category of crystalline aluminosilicates. In this context, when the T atoms are substantially atoms of aluminum (Al) and silicon (Si), it does not only mean that atoms of aluminum (Al) and silicon (Si) are the only T atoms, but also allows the presence of T atoms other than atoms of aluminum (Al) and silicon (Si), as long as the advantageous effects of the present invention are delivered.

A "zeotype" is a compound having an ordered structure in which T atoms are arranged with oxygen interposed therebetween, and is a compound in which the T atoms include at least atoms other than metal or metalloid atoms. Examples of zeotypes include complex phosphorus compounds, which contain phosphorus (P) as T atoms, such as aluminophosphates (AlPOs) and silicoaluminophosphates (SAPOs).

The "ordered structure (Hereinafter also referred to as "framework structure.")" in the context of a zeolite or zeotype refers to a framework structure identified by a framework type code assigned by the Structure Commission of the International Zeolite Association (Hereinafter also referred to simply as "framework code.") and to a framework structure having a polymorph listed on the website of the International Zeolite Association, "http://www.iza-structure.org/databases/" (hereinafter also referred to as "intergrowth structure"). For example, an "MFI zeolite" is a zeolite having a framework structure identified by the framework code "MFI." Furthermore, a "ZSM-48 zeolite," for example, is a zeolite having an intergrowth structure composed of intergrown crystals formed by polymorph ZSM-48_A and polymorph ZSM-48_B. Intergrowth structures in this embodiment are not particularly limited in terms of the ratio between polymorphs (Hereinafter also referred to as "intergrowth ratio.") but include intergrowth structures of any intergrowth ratio.

The framework structure (intergrowth structure) of a zeolite can be identified by comparison with the XRD patterns of the individual framework structures presented in Zeolite Framework on the website of the IZA Structure Commission, http://www.iza-structure.org/databases/ (Hereinafter also referred to as "reference patterns."). With respect to structures of zeolites, the terms of framework structure (intergrowth structure), crystal structure and crystal phase are used interchangeably with each other.

A "zeolite having an oxygen ten-membered ring structure" is a zeolite that includes, as rings (pores) formed by the T atoms and oxygen atoms constituting its framework structure, rings in which the number of oxygen atoms is ten (hereinafter also referred to as "oxygen ten-membered ring"). A "zeolite having an oxygen ten-membered ring structure" may be a zeolite in which the oxygen ten-membered rings are the only rings formed in its framework structure, or may be a zeolite that includes rings other than oxygen ten-membered rings in addition to the oxygen ten-membered rings. It should be noted that hereinafter, a zeolite having an oxygen ten-membered ring structure is also referred to as oxygen ten-membered ring zeolite.

"Xylene" is a general term for p-xylene, o-xylene and m-xylene. Unless otherwise specified, it represents one or more selected from the group of p-xylene, o-xylene and m-xylene.

A chemical formulation in this embodiment, such as the SiO₂/Al₂O₃ ratio, the specified element content and the additional element content, each of which will be described later, can be determined by inductively coupled plasma atomic emission spectroscopy (ICP-AES) using a commonly used inductively coupled plasma emission spectrometer (ICP system) (e.g., OPTIMA 5300DV, manufactured by PerkinElmer, Inc.). It should be noted that the composition analysis can be performed using a sample solution obtained by dissolving a sample in an aqueous solution mixture of hydrofluoric acid and nitric acid.

In the following, a zeolite according to the present disclosure will be described by presenting an example of an embodiment. The present disclosure encompasses any combination of the configurations and parameters disclosed herein and also encompasses any combination of the upper limits and lower limits of values disclosed herein.

A zeolite according to this embodiment is an oxygen ten-membered ring zeolite and contains one or more elements selected from the group of magnesium, calcium, titanium, boron and phosphorus. In addition, the total pore volume is 0.21 cm³/g or less.

The zeolite according to this embodiment can be any oxygen ten-membered ring zeolite. When considered from the viewpoint of further increasing p-xylene selectivity, however, it is preferred that the zeolite be an MFI zeolite, an MEL zeolite, a TON zeolite, an STF zeolite, an MTT zeolite, an MWW zeolite or a ZSM-48 zeolite, more preferably an MFI zeolite or an MEL zeolite, even more preferably an MFI zeolite.

In the zeolite according to this embodiment, the T atoms, which constitute the framework structure, only need be at least one of metal atoms or metalloid atoms and are not particularly limited. When considered from the viewpoint of further increasing p-xylene selectivity, however, it is preferred that the T atoms be substantially atoms of aluminum (Al) and silicon (Si). In other words, the zeolite according to this embodiment is preferably a crystalline aluminosilicate having an oxygen ten-membered ring structure.

The zeolite according to this embodiment contains one or more elements selected from the group of magnesium, calcium, titanium, boron and phosphorus (hereinafter also referred to as "specified elements"). Among magnesium, calcium, titanium, boron and phosphorus, the specified elements contained in the zeolite according to this embodiment are particularly preferably at least one of magnesium, calcium or phosphorus, more preferably at least one of magnesium or calcium, even more preferably magnesium, for further increased p-xylene selectivity.

The state of the specified elements contained in the zeolite according to this embodiment is not particularly limited. For example, however, the elements can be in a state of compounds (e.g., oxides), metals (or simple substances), ions, an alloy or two or more of these states.

In the zeolite according to this embodiment, the amount of the specified elements relative to 100% by mass as a dry mass of the zeolite according to this embodiment (hereinafter also referred to as "specified element content") is not particularly limited. From the viewpoint of further increasing p-xylene selectivity, however, it is preferred that the specified element content be 2% by mass or more, more preferably 4% by mass or more, even more preferably 6% by mass or more. The specified element content of the zeolite according to this embodiment, furthermore, is preferably 12% by mass or less, more preferably 10% by mass or less, for further increased p-xylene selectivity. The upper limit and lower limit of the specified element content can be any combination of the upper limits and lower limits specified above, but preferably, the upper limit and lower limit are 2% by mass or more and 12% by mass or less, more preferably 4% by mass or more and 12% by mass or less, even more preferably 6% by mass or more and 10% by mass or less. In this embodiment, the dry mass is the mass of the zeolite after 60 minutes of heat treatment at 600°C in an air atmosphere.

It should be noted that when two or more specified elements are contained, the above specified element content represents the total amount of the two or more elements, and when one specified element is contained, the specified element content represents the amount of this single element.

The form in which the specified elements are contained in the zeolite according to this embodiment is not particularly limited. When considered from the viewpoint of further increasing p-xylene selectivity, however, it is preferred that the specified elements be in a form in which they are supported on the zeolite. It should be noted that in this embodiment, when a specified element is contained, it means that the specified element is included in the zeolite, and the specified element may be included in any state and at any site. When a specified element is supported, on the other hand, it means that the specified element is included in the zeolite as a component other than the T atoms. An example of a form in which the specified elements are supported is a form in which the specified elements are supported on at least one of the external surface of the zeolite (the surface of the zeolite excluding the internal surfaces of pores) or the internal surfaces of pores. When considered from the viewpoint of further increasing p-xylene selectivity, it is preferred that the form in which the specified elements are supported be a form in which the specified elements are supported at least on the external surface of the zeolite, more preferably a form in which the specified elements are supported on both the external surface of the zeolite and the internal surfaces of pores.

The reason why p-xylene selectivity further increases when the specified elements are supported at least on the external surface of the zeolite is unclear. The inventors, however, believe that the specified elements supported on the external surface of the zeolite interact with acid sites on the external surface of the zeolite, inhibiting progress of the alcohol reforming reaction by acid sites on the external surface of the zeolite. As a result, the percentage of xylene produced at acid sites on the external surface of the zeolite is reduced, and the percentage of xylene produced at acid sites inside the zeolite is increased. Xylene produced at acid sites inside the zeolite is discharged through pores (rings) formed in the zeolite. With the zeolite according to this embodiment, however, o-xylene and m-xylene are presumably unlikely to be discharged from pores, whereas p-xylene is likely to be discharged from pores, as will be described later. When the percentage of xylene produced at acid sites inside the zeolite increases, therefore, it results in an increase in the percentage of xylene discharged through pores (rings), which preferentially discharge p-xylene. As a result, the inventors believe, p-xylene selectivity further improves.

The zeolite according to this embodiment has a total pore volume of 0.21 cm³/g or less. The total pore volume is the volume of pores per unit mass of the zeolite. The total pore volume of the zeolite according to this embodiment can be calculated by determining the amount of adsorbed nitrogen V (cm³) at an equilibrium relative pressure (Hereinafter also referred to as "p/p₀.") of 0.990 from a nitrogen adsorption-desorption isotherm of the zeolite and substituting the determined amount V into formula (1) below. $Total pore volume \left({cm}^{3}/g\right) = V \times 1.547 \times {10}^{- 3}$

The nitrogen adsorption-desorption isotherm used to determine the amount of adsorbed nitrogen V in formula (1) above, furthermore, can be obtained by the volumetric method, in which nitrogen gas is adsorbed onto the zeolite at varied pressures and then quantified. The measurement by the volumetric method can be performed using a commonly used nitrogen adsorption analyzer (e.g., BELSORP-mini II, manufactured by MicrotracBEL Corporation), and the measurement conditions can be the following conditions.
Measurement temperature: -196°C
Pretreatment of zeolite: 2 hours of vacuum drying at 350°C

For the zeolite according to this embodiment, the lower limit of the total pore volume can be any value greater than 0 cm³/g. When considered from the viewpoint of further increasing p-xylene selectivity, however, it is preferred that the total pore volume be 0.05 cm³/g or more, more preferably 0.09 cm³/g or more. That is, the total pore volume of the zeolite according to this embodiment is preferably 0.05 cm³/g or more and 0.21 cm³/g or less, more preferably 0.09 cm³/g or more and 0.21 cm³/g or less, for further increased p-xylene selectivity.

In the zeolite according to this embodiment, the molar ratio of silica to alumina (Hereinafter also referred to as "SiO₂/Al₂O₃ ratio.") is not particularly limited. From the viewpoint of further increasing p-xylene selectivity, however, it is preferred that this ratio be 250 or less, more preferably 200 or less, even more preferably 100 or less. In the zeolite according to this embodiment, furthermore, the SiO₂/Al₂O₃ ratio is preferably 10 or greater, more preferably 20 or greater, even more preferably 30 or greater, for further increased p-xylene selectivity. The upper limit and lower limit of the SiO₂/Al₂O₃ ratio can be any combination of the upper limits and lower limits specified above, but preferably, the upper limit and lower limit are 10 or greater and 250 or less, more preferably 10 or greater and 200 or less, still more preferably 10 or greater and 100 or less, particularly preferably 20 or greater and 100 or less, most preferably 30 or greater and 100 or less, for further increased p-xylene selectivity.

The zeolite according to this embodiment may further contain, in addition to the specified elements described above, one or more additional elements different from the specified elements (hereinafter also referred to simply as "additional elements"). The additional elements that can be contained in the zeolite according to this embodiment is preferably one or more elements selected from the group of silver, zinc, gallium and iron. By allowing the zeolite according to this embodiment to contain such additional elements in addition to the specified elements, p-xylene selectivity can be increased, and the total yield of benzene, xylene and toluene (hereinafter also referred to as "BTX yield"), which are useful as chemical source materials, can also be increased. It should be noted that the BTX yield can be determined by multiplying alcohol conversion (%) by the ratio of the total number of moles (mol) of carbon atoms contained in benzene, xylene and toluene in the reaction product obtained by alcohol reforming to the total number of moles (mol) of carbon atoms contained in the reaction product (all components contained in the reaction product).

When the zeolite according to this embodiment contains additional elements, the specified elements and additional elements contained can be in any combination. When considered from the viewpoint of further increasing the BTX yield while increasing p-xylene selectivity, however, it is preferred that the combination be a combination of one or more specified elements selected from the group of magnesium, calcium and phosphorus and one or more additional elements selected from the group of zinc and gallium, more preferably a combination of one or more specified elements selected from the group of magnesium and calcium and one or more additional elements selected from the group of zinc and gallium, still more preferably a combination of one or more specified elements selected from the group of magnesium and calcium and zinc (an additional element), particularly preferably a combination of magnesium (a specified element) and zinc (an additional element).

The state of the additional elements that can be contained in the zeolite according to this embodiment is not particularly limited. For example, however, the elements can be in a state of compounds (e.g., oxides), metals (or simple substances), ions, an alloy or two or more of these states.

In the zeolite according to this embodiment, the amount of the additional elements relative to 100% by mass as a dry mass of the zeolite according to this embodiment (hereinafter also referred to as "additional element content") is not particularly limited. When considered from the viewpoint of further increasing the BTX yield while increasing p-xylene selectivity, however, it is preferred that the additional element content be 0.1% by mass or more, more preferably 0.3% by mass or more, even more preferably 0.5% by mass or more. The additional element content of the zeolite according to this embodiment, furthermore, is preferably 12% by mass or less, more preferably 10% by mass or less, even more preferably 3% by mass or less, for a further increased BTX yield combined with increased p-xylene selectivity. The upper limit and lower limit of the additional element content can be any combination of the upper limits and lower limits specified above. When considered from the viewpoint of further increasing the BTX yield while increasing p-xylene selectivity, however, it is preferred that the upper limit and lower limit be 0.1% by mass or more and 12% by mass or less, more preferably 0.3% by mass or more and 10% by mass or less, still more preferably 0.3% by mass or more and 3% by mass or less, particularly preferably 0.5% by mass or more and 3% by mass or less.

It should be noted that when two or more additional elements are contained, the above additional element content represents the total amount of the two or more elements, and when one additional element is contained, the additional element content represents the amount of this single element.

The form in which the additional elements that can be contained in the zeolite according to this embodiment are contained is not particularly limited. When considered from the viewpoint of further increasing the BTX yield while increasing p-xylene selectivity, however, it is preferred that the additional elements be in a form in which they are supported on the zeolite. An example of a form in which additional elements are supported is a form in which the additional elements are supported on at least one of the external surface of the zeolite or the internal surfaces of pores. Preferably, the form of support is a form in which the additional elements are supported at least on both the external surface of the zeolite and the internal surfaces of pores, more preferably a form in which the additional elements are supported on the internal surfaces of pores in the zeolite.

The reason why the BTX yield further increases when additional elements are supported at least on the internal surfaces of pores in the zeolite is unclear. The inventors, however, believe that the additional elements supported on the internal surfaces of pores in the zeolite interact with acid sites on the internal surfaces of pores in the zeolite, promoting the reaction in which the alcohol is reformed into aromatic compounds by acid sites on the internal surfaces of pores in the zeolite. As a result, the percentage of BTX produced at acid sites on the internal surfaces of pores in the zeolite increases, and the BTX yield further improves.

As mentioned above, the zeolite according to this embodiment preferably has the specified elements and the additional elements supported on its external surface. A situation in which metal elements (metal elements as the specified elements and metal elements as the additional elements) are supported on the external surface of the zeolite can be confirmed by the presence of a peak having a peak top within the range of 1630 cm⁻¹ to 1650 cm⁻¹, inclusive (hereinafter also referred to as "p(1630 cm⁻¹ to 1650 cm⁻¹)") in a difference spectrum obtained by subtracting an IR spectrum of the zeolite before adsorption of 2,6-di-tert-butylpyridine (Hereinafter also referred to as "background spectrum.") from an IR spectrum of the zeolite with 2,6-di-tert-butylpyridine adsorbed thereonto (Hereinafter also referred to as "adsorption spectrum.") (Hereinafter also referred to as "difference spectrum.").

It should be noted that p(1630 cm⁻¹ to 1650 cm⁻¹) is a peak attributed to 2,6-di-tert-butylpyridine adsorbed on metal elements supported on the zeolite. Since 2,6-di-tert-butylpyridine cannot enter the interior of an oxygen ten-membered ring zeolite because of its molecular size, it can be said that p(1630 cm⁻¹ to 1650 cm⁻¹) in the difference spectrum is a peak attributed to 2,6-di-tert-butylpyridine adsorbed on metal elements supported on the external surface of the zeolite. p(1630 cm⁻¹ to 1650 cm⁻¹) preferably has a peak top within the range of 1635 cm⁻¹ to 1645 cm⁻¹, inclusive.

In the same situation, a peak having a peak top within the range of 1600 cm⁻¹ to 1620 cm⁻¹, inclusive (Hereinafter also referred to as "p(1600 cm⁻¹ to 1620 cm⁻¹)."), in the difference spectrum is a peak attributed to 2,6-di-tert-butylpyridine adsorbed on acid sites (proton acid sites) present on the external surface of the zeolite. The ratio of the maximum intensity (height intensity) of p(1630 cm⁻¹ to 1650 cm⁻¹) to the maximum intensity (height intensity) of p(1600 cm⁻¹ to 1620 cm⁻¹) in the difference spectrum (hereinafter also referred to as "IR peak ratio"), therefore, serves as a measure of the relative quantity of metal elements to the quantity of acid sites on the external surface of the zeolite. It should be noted that p(1600 cm⁻¹ to 1620 cm⁻¹) preferably has a peak top within 1605 cm⁻¹ to 1615 cm⁻¹, inclusive. Maximum intensity (height intensity), furthermore, refers to intensity at a peak top.

For the zeolite according to this embodiment, the IR peak ratio can be any value equal to or greater than 0 and is not particularly limited. When considered from the viewpoint of further increasing p-xylene selectivity, however, it is preferred that the IR peak ratio be 1.4 or greater, more preferably greater than 1.5, even more preferably 1.6 or greater. For the zeolite according to this embodiment, the IR peak ratio can be any value equal to or greater than 0 and is not particularly limited. When considered from the viewpoint of further increasing p-xylene selectivity, however, it is preferred that the IR peak ratio be 5.0 or less, more preferably 4.0 or less, even more preferably 3.6 or less. The upper limit and lower limit of the IR peak ratio can be any combination of the upper limits and lower limits specified above. When considered from the viewpoint of further increasing p-xylene selectivity, however, it is preferred that the upper limit and lower limit be 1.4 or greater and 5.0 or less, more preferably greater than 1.5 and 5.0 or less, still more preferably greater than 1.5 and 4.0 or less, particularly preferably 1.6 or greater and 4.0 or less, most preferably 1.6 or greater and 3.6 or less.

The reason why p-xylene selectivity further increases as a result of the IR peak ratio falling within these ranges is unclear. The inventors, however, presume that when the IR peak ratio falls within these ranges, it is more likely that metal elements and acid sites on the external surface of the zeolite interact. As a result, the percentage of xylene produced at acid sites inside the zeolite further increases, and p-xylene selectivity further increases.

The background spectrum and the adsorption spectrum can be measured using an FT-IR analyzer (e.g., FT/IR-6600, manufactured by JASCO Corporation). In the measurement of the background spectrum, a measurement sample (zeolite) is press-molded into a disk shape (approximately 10 mg/cm²), and the resulting disk is loaded into a heated transmission cell and pretreated under a vacuum at 450°C for 2 hours. After the pretreatment, the temperature is lowered to 200°C under vacuum conditions, and the background spectrum is measured under the measurement conditions specified below. In the measurement of the adsorption spectrum, for example, 2,6-di-tert-butylpyridine is introduced into the heated transmission cell after the measurement of the background spectrum, and the 2,6-di-tert-butylpyridine and the measurement sample are brought into contact under conditions of 200°C, 30 minutes and 100 Pa. Thereafter, the heated transmission cell is treated under a vacuum at 200°C for 30 minutes, whereby excess 2,6-di-tert-butylpyridine is removed. Then the adsorption spectrum is measured under the measurement conditions specified below.
Measurement method: heated transmission
Measurement temperature: 200°C
Measurement wavenumber range: 800 to 4000 cm-1
Resolution: 2 cm-1
Number of scans: 128
Measurement atmosphere: vacuum

The zeolite according to this embodiment preferably contains no structure-directing agent (Hereinafter also referred to as "SDA.") derived from a source material for its manufacture (i.e., it is preferred that the SDA content of the zeolite according to this embodiment be 0% by mass) because in that case it is more likely that p-xylene selectivity further improves. The zeolite according to this embodiment, however, may contain an SDA, as long as the object of the present invention can be achieved. The SDA content of the zeolite according to this embodiment can be, for example, 0% by mass or more and 10% by mass or less, more than 0% by mass and 5% by mass or less or 1% by mass or more and 0.5% by mass or less relative to 100% by mass of the zeolite according to this embodiment. It should be noted that when an amount is 0% by mass or 0 mol% in this embodiment, it means that substantially none of the component is contained. When substantially none of a component is contained, it means that the component is not detected (below the limit of measurement).

The zeolite according to this embodiment described above can be used for the manufacture of xylene by alcohol reforming and can be used as a catalyst for alcohol reforming that reforms alcohols into xylene. The catalyst for alcohol reforming contains at least a zeolite according to this embodiment. By using a zeolite according to this embodiment in the manufacture of xylene by alcohol reforming, the percentage of p-xylene relative to the xylene manufactured (p-xylene selectivity) can be increased. It should be noted that in a method for manufacturing xylene by alcohol reforming, aromatic hydrocarbons other than xylene (e.g., benzene and toluene) may be co-produced in addition to xylene.

A method for manufacturing xylene by alcohol reforming includes a step of bringing a zeolite according to this embodiment and at least one fluid containing at least one alcohol (hereinafter also referred to as "alcohol-containing fluid") into contact (Hereinafter also referred to as "contact step."). An example of a specific method for bringing the zeolite according to this embodiment and the alcohol-containing fluid into contact is the method of packing the zeolite according to this embodiment into a fixed-phase flow reactor tube to form a packed bed and passing the alcohol-containing fluid through this packed bed.

In the method for manufacturing xylene by alcohol reforming, the alcohol reforming reaction proceeds upon contact of the zeolite according to this embodiment with the alcohol-containing fluid. The contact conditions under which the zeolite according to this embodiment and the alcohol-containing fluid are brought into contact, therefore, are not particularly limited. An example of preferred contact conditions are the conditions described below.

The temperature at which the zeolite according to this embodiment and the alcohol-containing fluid come into contact is preferably 300°C or above, more preferably 350°C or above, for further increased p-xylene selectivity. The temperature at which the zeolite according to this embodiment and the alcohol-containing fluid come into contact, furthermore, is preferably 550°C or below, more preferably 500°C or below, for further increased p-xylene selectivity. The upper limit and lower limit of the contact temperature can be any combination of the upper limits and lower limits specified above. When considered from the viewpoint of further increasing p-xylene selectivity, however, it is preferred that the upper limit and lower limit be 300°C or above and 550°C or below, more preferably 350°C or above and 550°C or below, even more preferably 350°C or above and 500°C or below.

The pressure (gauge pressure) at which the zeolite according to this embodiment and the alcohol-containing fluid are brought into contact is preferably 0.05 Mpa or more, more preferably 0.1 Mpa or more, for further increased p-xylene selectivity. The pressure (gauge pressure) at which the zeolite according to this embodiment and the alcohol-containing fluid are brought into contact, furthermore, is preferably 1 Mpa or less, more preferably 0.5 Mpa or less, for further increased p-xylene selectivity. The upper limit and lower limit of the contact pressure (gauge pressure) can be any combination of the upper limits and lower limits specified above. When considered from the viewpoint of further increasing p-xylene selectivity, however, it is preferred that the upper limit and lower limit be 0.05 Mpa or more and 1 Mpa or less, more preferably 0.05 Mpa or more and 0.5 Mpa or less, even more preferably 0.1 Mpa or more and 0.5 Mpa or less. It should be noted that the gauge pressure is a pressure measured by taking atmospheric pressure as 0 MPa.

The weight hourly space velocity (WHSV) of the alcohol in the alcohol-containing fluid that is brought into contact with the zeolite according to this embodiment is preferably 0.05 hr⁻¹ or more, more preferably 0.5 hr⁻¹ or more, for further increased p-xylene selectivity. The weight hourly space velocity (WHSV) of the alcohol in the alcohol-containing fluid that is brought into contact with the zeolite according to this embodiment, furthermore, is preferably 10 hr⁻¹ or less, more preferably 5 hr⁻¹ or less, for further increased p-xylene selectivity. The upper limit and lower limit of the weight hourly space velocity (WHSV) of the alcohol can be any combination of the upper limits and lower limits specified above. When considered from the viewpoint of further increasing p-xylene selectivity, however, it is preferred that the upper limit and lower limit be 0.05 hr⁻¹ or more and 10 hr⁻¹ or less, more preferably 0.05 hr⁻¹ or more and 5 hr⁻¹ or less, even more preferably 0.5 hr⁻¹ or more and 5 hr⁻¹ or less. It should be noted that the WHSV is a parameter representing hourly supply of the alcohol (supply of the alcohol on a liquid basis) per unit mass of the zeolite ([g (zeolite)]/[g (alcohol)/h] (= [hr⁻¹])).

The duration of contact between the zeolite according to this embodiment and the alcohol-containing fluid can be set as appropriate according to the desired amount of p-xylene obtained.

The alcohol-containing fluid used in the contact step may be a fluid consisting solely of the alcohol or may be a fluid containing one or more components other than alcohols in addition to the alcohol. The components other than alcohols can be, for example, one or more components selected from the group consisting of nitrogen, helium, argon and hydrogen.

The alcohol contained in the alcohol-containing fluid can be, for example, one or more selected from the group consisting of methanol, ethanol, butanol and isobutanol. When considered from the viewpoint of further increasing p-xylene selectivity, it is preferred that the alcohol be methanol.

The alcohol-containing fluid that is brought into contact with the zeolite according to this embodiment may be one or more selected from the group of a liquid, a gas and a fluid mixture of a liquid and a gas. When considered from the viewpoint of further increasing p-xylene selectivity, however, it is preferred that the alcohol-containing fluid be a gas.

In the contact step, the zeolite according to this embodiment that is brought into contact with the alcohol-containing fluid may be a zeolite according to this embodiment that has undergone activation treatment. The activation treatment of the zeolite according to this embodiment can be an activation treatment known in the related art, and the method therefor is not limited. An example of a specific activation treatment is an activation treatment in which nitrogen gas is passed through the zeolite according to this embodiment under the following conditions.
Nitrogen gas flow rate: 5 mL/min or more and 100 mL/min or less
Reaction temperature: 400°C or above and 600°C or below
Reaction pressure: 0.05 Mpa or more and 1 Mpa or less (gauge pressure)
Duration of reaction: 0.5 hours or more and 50 hours or less

The zeolite according to this embodiment in the contact step may be shaped in a specific shape. Examples of methods for shaping the zeolite according to this embodiment include tumbling granulation, press molding, extrusion molding, injection molding, casting molding and sheet molding. Examples of shapes of the shaped zeolite include a spherical shape, a substantially spherical shape, an elliptical shape, a disk shape, a columnar shape, a polyhedral shape, an irregular shape and a petal shape. It should be noted that the zeolite according to this embodiment may be shaped together with at least one binder to form a shaped article containing the binder and the zeolite. By passing the alcohol-containing fluid through such a shaped article, the zeolite according to this embodiment contained in the shaped article and the alcohol-containing fluid may be brought into contact. The binder can be, for example, at least one selected from the group of silica, alumina other than γ-alumina, kaolin, attapulgite, montmorillonite, bentonite and sepiolite.

For the zeolite according to this embodiment, it is preferred that p-xylene selectivity when the zeolite is subjected to the treatment of bringing a gas mixture of nitrogen and methanol into contact with it under conditions of a reaction temperature of 420°C, a reaction pressure (gauge pressure) of 0.2 MPa, a duration of reaction of 6 hours and a methanol weight hourly space velocity of 1.0 Hr⁻¹ (hereinafter also referred to as "methanol contact treatment (420°C)") (hereinafter also referred to as "p-xylene selectivity (420°C)") be 35% or more, preferably 40% or more, more preferably 60% or more, although not particularly limited. The upper limit of the p-xylene selectivity (420°C) is not particularly limited, but for example, the p-xylene selectivity (420°C) is 100% or less or 99.9% or less. The upper limit and lower limit of the p-xylene selectivity (420°C) can be any combination of the upper limits and lower limits specified above, but preferably, the p-xylene selectivity (420°C) is 35% or more and 100% or less, more preferably 40% or more and 100% or less, even more preferably 40% or more and 99.9% or less, particularly preferably 60% or more and 99.9% or less.

The p-xylene selectivity (420°C) can be determined by dividing the mass of p-xylene in the product produced by carrying out the methanol contact treatment (420°C) by the total mass of xylene (p-xylene, o-xylene and m-xylene) in the product and expressing the quotient as a percentage. The zeolite according to this embodiment that is subjected to the methanol contact treatment (420°C), furthermore, can be a zeolite that has undergone a treatment in which nitrogen gas at 50 mL/min is passed through the zeolite for 1 hour under conditions of a temperature of 450°C and 0.2 MPa (gauge pressure) (activation treatment).

For the zeolite according to this embodiment, it is preferred that the BTX yield when the zeolite is subjected to the methanol contact treatment (420°C) (hereinafter also referred to as "BTX yield (420°C)") be 3% or more, more preferably 15% or more, even more preferably 25% or more, although not particularly limited. The upper limit of the BTX yield (420°C) is not particularly limited, but for example, the BTX yield (420°C) is 100% or less or 70% or less. The upper limit and lower limit of the BTX yield (420°C) can be any combination of the upper limits and lower limits specified above, but preferably, the upper limit and lower limit are 3% or more and 100% or less, more preferably 15% or more and 70% or less, even more preferably 25% or more and 70% or less.

The BTX yield (420°C) can be determined by dividing the total mass of benzene, xylene (p-xylene, o-xylene and m-xylene) and toluene in the product produced by carrying out the methanol contact treatment (420°C) by the mass of the product (the total mass of all components in the product) and expressing the quotient as a percentage.

It should be noted that the zeolite according to this embodiment, which reforms alcohols into benzene, xylene and toluene, can also be used to co-produce benzene, xylene and toluene by alcohol reforming. It should be noted that a method for co-producing benzene, xylene and toluene using a zeolite according to this embodiment includes, like the method for manufacturing xylene using a zeolite according to this embodiment, a step of bringing the zeolite according to this embodiment and at least one alcohol-containing fluid into contact (contact step). The contact step in the method for co-producing benzene, xylene and toluene is the same step as the contact step in the method for manufacturing xylene, and thus detailed description thereof is omitted.

A method for manufacturing a zeolite according to this embodiment will now be described.

A method for manufacturing a zeolite according to this embodiment includes a step of allowing an oxygen ten-membered ring zeolite to contain the specified elements (hereinafter also referred to as "specified element inclusion step").

In the specified element inclusion step, the method for allowing the oxygen ten-membered ring zeolite to contain the specified elements is not particularly limited, but preferably, a contact treatment in which a solution containing the specified elements (hereinafter also referred to as "specified element solution") and the oxygen ten-membered ring zeolite are brought into contact (hereinafter also referred to as "specified element contact treatment") is used.

When the oxygen ten-membered ring zeolite is allowed to contain the specified elements through a specified element contact treatment, the total pore volume of the oxygen ten-membered ring zeolite may decrease, depending on treatment conditions. By allowing the oxygen ten-membered ring zeolite to contain the specified elements by a specified element contact treatment, therefore, a zeolite having a total pore volume of 0.21 cm³/g or less (i.e., a zeolite according to this embodiment) can be obtained depending on treatment conditions, even if the total pore volume of the oxygen ten-membered ring zeolite before contact with the specified element solution exceeds 0.21 cm³/g. The reason why the total pore volume of an oxygen ten-membered ring zeolite decreases through a specified element contact treatment is unclear. Presumably, however, when a specified element contact treatment is performed under specific conditions, the specified elements are more likely to reach not only the surface (external surface) of the oxygen ten-membered ring zeolite but also the interior, resulting in a decrease in total pore volume.

In the specified element contact treatment, the oxygen ten-membered ring zeolite that is brought into contact with the specified element solution preferably has a total pore volume of 0.35 cm³/g or less, more preferably 0.28 cm³/g or less, even more preferably 0.21 cm³/g or less, particularly preferably 0.19 cm³/g or less, because in that case a zeolite according to this embodiment is more likely to be obtained. The total pore volume of the oxygen ten-membered ring zeolite that is brought into contact with the specified element solution is not particularly limited, as long as it is greater than 0 cm³/g. For example, however, it can be 0.05 cm³/g or more, 0.07 cm³/g or more or 0.15 cm³/g or more. The upper limit and lower limit of the total pore volume of the oxygen ten-membered ring zeolite that is brought into contact with the specified element solution can be any combination of the upper limits and lower limits specified above, but preferably, the upper limit and lower limit are 0.05 cm³/g or more and 0.35 cm³/g or less, more preferably 0.05 cm³/g or more and 0.28 cm³/g or less, even more preferably 0.07 cm³/g or more and 0.28 cm³/g or less, particularly preferably 0.15 cm³/g or more and 0.28 cm³/g or less, because in that case a zeolite according to this embodiment is more likely to be obtained.

The oxygen ten-membered ring zeolite that is brought into contact with the specified element solution is preferably an oxygen ten-membered ring zeolite to which SDA removal treatment, which will be described later, has been applied, more preferably an oxygen ten-membered ring zeolite containing substantially no SDA. An oxygen ten-membered ring zeolite to which SDA removal treatment has been applied is more likely to undergo a decrease in total pore volume through the specified element contact treatment compared with an oxygen ten-membered ring zeolite without SDA removal treatment. With such a zeolite, therefore, it is more likely that a zeolite having a total pore volume of 0.21 cm³/g or less (i.e., a zeolite according to this embodiment) is obtained.

For the oxygen ten-membered ring zeolite that is brought into contact with the specified element solution, its framework structure, the types of T atoms constituting the framework structure and the SiO₂/Al₂O₃ ratio are not particularly limited; they can be set as appropriate according to the framework structure, the types of T atoms constituting the framework structure and the SiO₂/Al₂O₃ ratio of the zeolite according to this embodiment that is to be manufactured.

The specified element solution that is brought into contact with the oxygen ten-membered ring zeolite is a solution containing the specified elements and at least one solvent. The specified elements contained in the specified element solution may be contained in the form of at least one compound containing a specified element. The compound containing a specified element can be, for example, at least one or more compounds selected from the group consisting of acetates, nitrates, ammonium salts, sulfates, hydroxides and chlorides of the specified elements. Of these, it is particularly preferred that the compound be at least one of an acetate, a nitrate or an ammonium salt of a specified element. Specifically, the compound containing a specified element can be, for example, at least one or more compounds selected from the group consisting of magnesium acetate, magnesium nitrate, magnesium chloride, magnesium sulfate, calcium nitrate, calcium chloride, calcium sulfate, phosphoric acid, ammonium hydrogen phosphate, diammonium hydrogen phosphate, titanium chloride, titanium sulfate, titanyl sulfate, boric acid and ammonium tetraborate, and preferably is at least one of magnesium acetate, calcium nitrate or diammonium hydrogen phosphate. The solvent contained in the specified element solution can be, for example, at least one of water or an alcohol, and preferably is water.

The concentration of the specified elements in the specified element solution and the amount of the specified element solution that is brought into contact with the oxygen ten-membered ring zeolite are not particularly limited; they can be adjusted as appropriate such that the total pore volume of the oxygen ten-membered ring zeolite resulting from contact with the specified element solution is 0.21 cm³/g or less. As the concentration of the specified elements in the specified element solution decreases, however, it tends to be less likely that the total pore volume of the oxygen ten-membered ring zeolite subjected to the specified element contact treatment decreases. When the total pore volume of the oxygen ten-membered ring zeolite before contact with the specified element solution exceeds 0.21 cm³/g, therefore, it is preferred to adjust the concentration of the specified elements in the specified element solution as appropriate considering the above tendency, and it is more preferred to adjust it to a concentration at which the specified element content of the oxygen ten-membered ring zeolite after contact with the specified element solution is 3% by mass or more.

The conditions for contact between the oxygen ten-membered ring zeolite and the specified element solution are not particularly limited; they can be adjusted as appropriate such that the total pore volume of the oxygen ten-membered ring zeolite resulting from contact with the specified element solution is 0.21 cm³/g or less. For example, the duration of contact between the oxygen ten-membered ring zeolite and the specified element solution can be 0.1 minutes or more and 48 hours or less. For example, furthermore, the temperature at which the oxygen ten-membered ring zeolite and the specified element solution come into contact can be 5°C or above and 100°C or below. Moreover, for example, the pressure at which the oxygen ten-membered ring zeolite and the specified element solution come into contact can be 0 Mpa or more and 1 Mpa or less (gauge pressure).

As a result of the contact between the oxygen ten-membered ring zeolite and the specified element solution, the specified elements come to be contained in the oxygen ten-membered ring zeolite. Thereby, a zeolite according to this embodiment can be manufactured. It should be noted that the oxygen ten-membered ring zeolite after contact with the specified element solution may be directly used as a zeolite according to this embodiment, but it may be used as a zeolite according to this embodiment after at least one treatment selected from drying treatment and firing treatment.

Drying treatment is a treatment in which water is removed from the oxygen ten-membered ring zeolite after contact with the specified element solution. The drying conditions are not particularly limited; they only need to be such that water can be removed from the oxygen ten-membered ring zeolite. For example, however, the conditions can be drying for 0.5 hours or more and 48 hours or less at 60°C or above and 180°C or below in an air atmosphere.

Firing treatment is a treatment in which the oxygen ten-membered ring zeolite after contact with the specified element solution or the oxygen ten-membered ring zeolite after drying treatment is fired. The firing conditions can be, for example, the following conditions.
Firing temperature: 350°C or above or 450°C or above, and
   700°C or below or 600°C or below
Duration of firing: 0.5 hours or more or 1 hour or more, and
   24 hours or less or 12 hours or less
Firing atmosphere: air atmosphere

As mentioned above, a zeolite according to this embodiment may contain one or more additional elements different from the specified elements in addition to the specified elements. A zeolite according to this embodiment that further contains additional elements in addition to the specified elements can be manufactured by a manufacturing method that includes the specified element inclusion step described above and a step of allowing the oxygen ten-membered ring zeolite to contain the additional elements (hereinafter also referred to as "additional element inclusion step").

In the additional element inclusion step, the method for allowing the oxygen ten-membered ring zeolite to contain the additional elements is not particularly limited. For example, however, a contact treatment in which a solution containing the additional elements (hereinafter also referred to as "additional element solution") and the oxygen ten-membered ring zeolite are brought into contact (hereinafter also referred to as "additional element contact treatment") can be used.

The additional element solution used in the additional element contact treatment is a solution containing the additional elements and at least one solvent. The additional elements contained in the additional element solution may be contained in the form of at least one compound containing an additional element. The compound containing an additional element can be, for example, at least one or more compounds selected from the group consisting of nitrates, acetates, sulfates and chlorides of the additional elements, and preferably is nitrate(s). Specifically, the compound containing an additional element can be, for example, at least one or more compounds selected from the group consisting of gallium nitrate, gallium chloride, zinc nitrate, zinc sulfate, zinc chloride, silver nitrate, iron(II) sulfate, iron(III) sulfate, iron chloride, iron oxalate, iron citrate and iron nitrate, and preferably is at least one of gallium nitrate or zinc nitrate. The solvent contained in the additional element solution can be, for example, at least one of water or an alcohol and preferably is water.

The additional element contact treatment is the same treatment as the specified element contact treatment described above except that the additional element solution is used instead of the specified element solution, and thus detailed description thereof is omitted.

In the method for manufacturing a zeolite according to this embodiment that further contains additional elements, the order of the specified element inclusion step and the additional element inclusion step is not particularly limited; the specified element inclusion step may be performed after the additional element inclusion step, or the additional element inclusion step may be performed after the specified element inclusion step.

In this case, when the specified element contact treatment is performed after the additional element contact treatment, the total pore volume of the oxygen ten-membered ring zeolite is more likely to decrease than when the additional element contact treatment is performed after the specified element contact treatment. In a manufacturing method in which the specified element contact treatment is performed after the additional element contact treatment, therefore, a zeolite having a total pore volume of 0.21 cm³/g or less (i.e., a zeolite according to this embodiment) is more likely to be manufactured, even if the total pore volume of the oxygen ten-membered ring zeolite before these treatments exceeds 0.21 cm³/g. In the method for manufacturing a zeolite according to this embodiment that further contains additional elements, therefore, it is preferred to perform a step of allowing an oxygen ten-membered ring zeolite to contain the additional elements through an additional element contact treatment (additional element inclusion step) first, and then a step of allowing the oxygen ten-membered ring zeolite containing the additional elements to contain the specified elements through a specified element contact treatment (specified element inclusion step).

The oxygen ten-membered ring zeolite used in the specified element inclusion step and the additional element inclusion step may be a commercially available zeolite or may be a zeolite obtained by crystallizing a composition containing an alumina source, a silica source, an organic structure-directing agent source, an alkali source and at least one type of water (hereinafter also referred to as "source composition").

In the following, a method for manufacturing an oxygen ten-membered ring zeolite in which a source composition is crystallized will be described.

The alumina source contained in the source composition is at least one compound containing aluminum (Al). The compound can be, for example, one or more selected from the group of aluminum isopropoxide, aluminum sulfate, aluminum chloride, aluminum hydroxide, pseudoboehmite, alumina sol and amorphous aluminosilicate, with amorphous aluminosilicate being preferred. It should be noted that a substance containing aluminum (Al) and silicon (Si), such as amorphous aluminosilicate, can be used not only as an alumina source but also as a silica source, which will be described below.

The silica source contained in the source composition is at least one compound containing silicon (Si). The compound can be, for example, one or more selected from the group of silica sol, fumed silica, colloidal silica, precipitated silica, sodium silicate, amorphous silicic acid and amorphous aluminosilicate, with amorphous aluminosilicate being preferred.

The organic structure-directing agent (Hereinafter also referred to as "SDA.") source can be any substance containing an SDA that facilitates formation of a framework structure including an oxygen ten-membered ring, and at least one known SDA that is known to facilitate formation of an oxygen ten-membered ring zeolite can be used. For example, SDA(s) that facilitate formation of an MFI zeolite can be at least one or more selected from the group consisting of normal butylamine, tetrapropylammonium bromide, diethylenetriamine and cyclohexylamine. Of these, it is particularly preferred that the SDA(s) be at least one or more selected from the group consisting of normal butylamine, diethylenetriamine and cyclohexylamine, more preferably normal butylamine. With normal butylamine, diethylenetriamine and cyclohexylamine, the total pore volume of the resulting oxygen ten-membered ring zeolite can be reduced to a greater extent compared with other SDAs.

The SDA source may be at least one salt of an SDA. For example, it can be one or more salts selected from chlorides, bromides, iodides and hydroxides.

The alkali source contained in the source composition is at least one compound containing an alkali metal element and can be, for example, at least one compound containing one or more alkali metal elements selected from the group of sodium, potassium, cesium and rubidium. The alkali source may be in the form of one or more salts selected from the group consisting of hydroxides, carbonates, chlorides, bromides, iodides and sulfates containing alkali metal element(s). A preferred alkali source can be, for example, at least one or more in the group consisting of sodium hydroxide, sodium carbonate, sodium chloride, sodium bromide, sodium iodide and sodium sulfate.

The water contained in the source composition can be, for example, distilled water, deionized water, purified water or two or more types selected therefrom. It should be noted that when a source material contained in the source composition other than water is a material containing water, for example as a hydrate, structural water or a solvent, the water contained in the source material other than water can be deemed as a type of water contained in the source composition.

The source composition may be composed solely of the alumina source, the silica source, the SDA source, the alkali source and water, but it may contain additional source materials other than these source materials.

The formulation of the source composition can be selected as appropriate, for example according to the framework structure of the oxygen ten-membered ring zeolite to be manufactured. An example of a preferred formulation, however, is the following molar formulation. It should be noted that each ratio in the following formulation is a molar (mol) ratio. SiO2 denotes the molar amount of silicon, Al₂O₃ denotes the molar amount of aluminum as alumina, H₂O denotes the molar amount of water, M denotes the molar amount of the alkali metal element(s) (total molar amount of alkali metal elements), SDA denotes the molar amount of the organic structure-directing agent, and OH⁻ denotes the molar amount of hydroxide ions.
SiO₂/Al₂O₃ ratio = 15 or greater, preferably 23 or greater, and
   500 or less, preferably 300 or less
SDA/SiO₂ ratio = 0.05 or greater, preferably 0.10 or greater, and
   0.50 or less, preferably 0.30 or less
M/SiO₂ ratio = 0.05 or greater, preferably 0.10 or greater, and
   0.15 or less, preferably 0.20 or less
H₂O/SiO₂ ratio = 5 or greater, preferably 10 or greater, and
   40 or less, preferably 20 or less
OH⁻/SiO₂ ratio = 0.05 or greater, preferably 0.10 or greater, and
   0.20 or less, preferably 0.15 or less

The source composition can be obtained by mixing the above-described alumina source, silica source, SDA source, alkali source, water and other source materials that are optionally contained.

The crystallization treatment of the source composition can be any treatment with which the source composition can be crystallized such that an oxygen ten-membered ring zeolite is obtained, and the treatment method therefor is not particularly limited. An example of a preferred method for the crystallization treatment is the method of subjecting the source composition to hydrothermal treatment. The hydrothermal treatment can be performed by, for example, placing the source composition in a hermetically sealed pressure-resistant container and heating the container. The conditions for the hydrothermal treatment can be, for example, the following conditions.
Reaction temperature: 90°C or above or 115°C or above, and
   200°C or below or 180°C or below
Duration of reaction: 10 hours or more or 20 hours or more, and
   72 hours or less or 48 hours or less
Reaction pressure: autogenous pressure

The crystallization treatment of the source composition may be performed after adding seed crystals to the source composition. The seed crystals are crystals of a zeolite having a function of promoting formation of the framework structure of the oxygen ten-membered ring zeolite to be manufactured. For example, they can be crystals of a zeolite having the same framework structure as the zeolite to be manufactured. The amount of seed crystals added can be, for example, 0.1% by mass or more and 10% by mass or less, and can also be 0.5% by mass or more and 3% by mass or less, relative to 100% by mass of the source composition (excluding the seed crystals).

It should be noted that to the oxygen ten-membered ring zeolite obtained through this crystallization treatment, one or more treatments selected from the group consisting of washing treatment, drying treatment, SDA removal treatment and ammonium treatment may be applied. Preferably, at least SDA removal treatment is applied. An oxygen ten-membered ring zeolite to which SDA removal treatment has been applied is more likely to undergo a decrease in total pore volume through the specified element contact treatment, and is more likely to have a total pore volume of 0.21 cm³/g or less after the specified element inclusion step. With SDA removal treatment, therefore, it is more likely that a zeolite according to this embodiment is manufactured.

Washing treatment is a step of washing the oxygen ten-membered ring zeolite. For example, in the washing treatment, the oxygen ten-membered ring zeolite can be washed with purified water.

Drying treatment is a treatment in which water is removed from the oxygen ten-membered ring zeolite. The conditions for the drying treatment are not particularly limited, as long as water can be removed from the oxygen ten-membered ring zeolite. For example, the drying temperature can be 100°C or above and 150°C or below. For example, furthermore, the duration of drying can be 2 hours or more and 20 hours or less. Moreover, for example, the atmosphere during drying can be air.

SDA removal treatment is a treatment in which SDAs contained in the oxygen ten-membered ring zeolite are removed. Typically, a zeolite crystallized using an SDA contains the SDA in its pores. Through inclusion of an SDA removal step, SDAs contained in the oxygen ten-membered ring zeolite can be removed.

SDA removal treatment can be performed by any method; the treatment only needs to be such that SDAs are removed. The method for removing SDAs can be, for example, at least one or more selected from the group consisting of liquid-phase treatment using an acidic aqueous solution, exchange treatment using a material such as a resin, and heat treatment (thermal decomposition). From the viewpoint of manufacturing efficiency, it is preferred that the SDA removal treatment be heat treatment (thermal decomposition). The conditions for the heat treatment (thermal decomposition) are not particularly limited, as long as SDAs are removed. For example, the heat treatment temperature can be 400°C or above and 800°C or below. For example, furthermore, the duration of heat treatment can be 1 hour or more and 5 hours or less. Moreover, for example, the atmosphere during heat treatment can be air.

Ammonium treatment is a treatment for removing alkali metals contained in the oxygen ten-membered ring zeolite. Ammonium treatment can be performed by a known method. For example, it can be performed by bringing an aqueous solution containing ammonium ions and the oxygen ten-membered ring zeolite into contact. Through the ammonium treatment, the cation type of the oxygen ten-membered ring zeolite becomes NH₄. It should be noted that the oxygen ten-membered ring zeolite after the ammonium treatment can be converted to a protonic (H⁺) cation type through firing treatment at 400°C or above and 700°C or below.

With a zeolite according to this embodiment as described above, p-xylene selectivity can be increased in the manufacture of xylene by alcohol reforming. The reason why a zeolite according to this embodiment increases p-xylene selectivity is unclear. The inventors, however, believe that a total pore volume of 0.21 cm³/g or less of the oxygen ten-membered ring zeolite combined with the presence of the specified elements in the oxygen ten-membered ring zeolite causes steric hindrance when xylene produced inside the oxygen ten-membered ring zeolite (at acid sites inside the zeolite) is discharged through pores. As a result, presumably, o-xylene and m-xylene, among the types of xylene produced inside the oxygen ten-membered ring zeolite, are unlikely to be discharged from pores, and only p-xylene, among the types of xylene produced in pores, is likely to be discharged out of the pores, resulting in increased p-xylene selectivity.

With a zeolite according to this embodiment, furthermore, p-xylene selectivity can be increased without using an expensive organic silicon like that used in PTL 1. A zeolite according to this embodiment, therefore, does not necessarily require equipment for detoxifying waste fluid containing organic silicon, and thus can be adapted to industrial processes at low cost.

### EXAMPLES

In the following, the present disclosure will be described more specifically by providing examples. The present disclosure, however, is not limited to the examples below in any way.

### (Identification of the Crystal Phase)

For a measurement sample (zeolite), an XRD measurement of the measurement sample was performed using a commonly used powder X-ray diffractometer (instrument name: Ultima IV, manufactured by Rigaku Corporation). The measurement conditions are as follows.
Accelerating current and voltage: 10 mA and 30 kV
X-ray source: CuKα radiation (λ = 1.54178 Å)
Measurement mode: continuous scanning
Scan condition: 2°/min
Measurement range: 2θ = 10° to 70°
Scattering slit: 1/3°
Divergence slit: 1/3°
Receiving slit: 0.3 mm
Filter: a Ni filter

The XRD pattern obtained was subjected to baseline correction and detection and intensity analysis of each XRD peak after correction using analysis software attached to the measuring instrument (product name: IGOR Pro 8, manufactured by WaveMetrics, Inc.). By comparing the corrected XRD pattern and a reference pattern, the crystal phase of the zeolite was identified.

### (Composition Analysis)

As a pretreatment, a measurement sample was heat-treated in an air atmosphere at 600°C for 60 minutes. The pretreated measurement sample was dissolved in an aqueous solution mixture of hydrofluoric acid and nitric acid, whereby a sample solution was prepared. Using a commonly used ICP system (instrument name: OPTIMA 5300DV, manufactured by PerkinElmer, Inc.), the sample solution was measured by inductively coupled plasma atomic emission spectroscopy (ICP-AES). From the measured values for aluminum (Al), silicon (Si), a specified element and an additional element, the SiO₂/Al₂O₃ ratio of the measurement sample, the percentage by mass of the specified element relative to the measurement sample (specified element content) and the percentage by mass of the additional element relative to the measurement sample (additional element content) were determined. It should be noted that the mass of the measurement sample used was the mass of the pretreated measurement sample.

### (Total Pore Volume)

A nitrogen adsorption-desorption isotherm of a measurement sample was obtained using a commonly used nitrogen adsorption analyzer (analyzer name: BELSORP-mini II, manufactured by MicrotracBEL Corporation). The nitrogen gas adsorption was performed using the volumetric method. The measurement conditions are as specified below.
Measurement temperature: -196°C
Pretreatment: vacuum drying at 350°C for 2 hours

The amount of adsorbed nitrogen gas V (cm³) at a p/p₀ of 0.990 was determined from the nitrogen adsorption-desorption isotherm. By substituting the amount V obtained into formula (1) above, the total pore volume was calculated.

### (IR Spectra)

The IR spectra were measured using an FT-IR analyzer (analyzer name: FT/IR-6600, manufactured by JASCO Corporation) equipped with a heated transmission cell (manufactured by Makuhari Rikagaku Garasu Inc.). The measurement sample was press-molded into a disk shape (approximately 10 mg/cm²) and pretreated in the heated transmission cell under a vacuum at 450°C for 2 hours. Thereafter, the temperature was lowered to 200°C under vacuum conditions, and the background spectrum was measured under the following measurement conditions.
Measurement method: heated transmission method
Measurement temperature: 200°C
Measurement wavenumber range: 800 to 4000 cm-1
Resolution: 2 cm-1
Number of scans: 128
Measurement atmosphere: vacuum

Subsequently, 2,6-di-tert-butylpyridine was introduced into the heated transmission cell and brought into contact with the measurement sample for 30 minutes at 200°C for 30 minutes at 100 Pa. Thereafter, the heated transmission cell was treated under a vacuum at 200°C for 30 minutes, whereby excess 2,6-di-tert-butylpyridine was removed, and then the adsorption spectrum was measured under the measurement conditions specified above. The background spectrum was subtracted from the obtained adsorption spectrum to give a difference spectrum. In the difference spectrum obtained, the maximum intensity (height intensity) of p(1600 cm⁻¹ to 1620 cm⁻¹) and the maximum intensity (height intensity) of p(1630 cm⁻¹ to 1650 cm⁻¹) were determined, and the IR peak ratio was determined.

### SYNTHESIS EXAMPLE 1

### (Synthesis of an MFI Zeolite)

Normal butylamine (hereinafter also referred to as "NBA"), a 48% by mass aqueous solution of sodium hydroxide, purified water and amorphous aluminosilicate (SiO₂/Al₂O₃ ratio: 50) were mixed to give a source composition having the following molar formulation.
SiO₂/Al₂O₃ ratio = 50
NBA/SiO₂ ratio = 0.10
Na/SiO₂ ratio = 0.10
OH⁻/SiO₂ ratio = 0.10
H₂O/SiO₂ ratio = 11

Seed crystals (MFI zeolite; SiO₂/Al₂O₃ molar ratio, 2400) were mixed into 55 g of the source composition such that the seed crystal content was 1% by mass relative to 100% by mass of the source composition, and the resulting mixture was packed in a hermetically sealed container having a capacity of 80 mL. After packing, the composition was allowed to react by hydrothermal treatment at 150°C for 36 hours and under autogenous pressure while the container was rotated at 55 rpm, whereby a crystallized material was obtained. The resulting crystallized material was collected after solid-liquid separation, washing with purified water and drying at 110°C in an air atmosphere. The crystallized material (Hereinafter also referred to as "the crystallized material of Synthesis Example 1.") consisted of a single phase of an MFI zeolite, in which the SiO₂/Al₂O₃ molar ratio was 48, and the total pore volume was 0.23 cm³/g.

Then the resulting crystallized material of Synthesis Example 1 (MFI zeolite) was fired at 600°C for 2 hours in an air atmosphere (subjected to SDA removal treatment). The fired MFI zeolite and an aqueous solution of ammonium chloride in which the NH₄Cl concentration was 20% by mass were mixed at 60°C such that the ratio by mass was 1:1, whereby the zeolite was converted to an MFI zeolite having an NH₄ cation type. This MFI zeolite was washed with purified water and dried at 110°C for 12 hours in an air atmosphere, whereby an MFI zeolite of this synthesis example was obtained.

The MFI zeolite of this synthesis example was a crystalline aluminosilicate having a SiO₂/Al₂O₃ molar ratio of 48 and a total pore volume of 0.25 cm³/g. The MFI zeolite of this synthesis example, furthermore, had an IR peak ratio of 0.1.

### SYNTHESIS EXAMPLE 2

Except that tetrapropylammonium bromide (hereinafter also referred to as "TPABr") was used instead of NBA and that the molar formulation was changed as follows, a source composition having the following molar formulation was obtained by the same method as in Synthesis Example 1.
SiO₂/Al₂O₃ ratio = 50
TPABr/SiO₂ ratio = 0.05
Na/SiO₂ ratio = 0.17
OH⁻/SiO₂ ratio = 0.17
H₂O/SiO₂ ratio = 10

Seed crystals (MFI zeolite; SiO₂/Al₂O₃ molar ratio, 50) were mixed into 55 g of the source composition such that the seed crystal content was 1% by mass relative to 100% by mass of the source composition, and the resulting mixture was packed in a hermetically sealed container having a capacity of 80 mL. After packing, the composition was allowed to react by hydrothermal treatment at 115°C for 84 hours and under autogenous pressure while the container was rotated at 55 rpm, whereby a crystallized material was obtained. The resulting crystallized material was collected after solid-liquid separation, washing with purified water and drying at 110°C in an air atmosphere. The crystallized material (hereinafter also referred to as "the crystallized material of Synthesis Example 2") consisted of a single phase of an MFI zeolite and was an MFI zeolite (crystalline aluminosilicate) having a SiO₂/Al₂O₃ molar ratio of 48.

Then the resulting crystallized material (MFI zeolite) of Synthesis Example 2 was fired, subjected to ion exchange, washed and dried under the same conditions as in the firing, ion exchange, washing and drying performed for the crystallized material of Synthesis Example 1, whereby an MFI zeolite of this synthesis example was obtained.

The MFI zeolite of this synthesis example was a crystalline aluminosilicate having a SiO₂/Al₂O₃ molar ratio of 48 and a total pore volume of 0.64 cm³/g. The MFI zeolite of this synthesis example, furthermore, had an IR peak ratio of 0.0.

### SYNTHESIS EXAMPLE 3

Except that amorphous aluminosilicate (SiO₂/Al₂O₃ ratio: 83) was used instead of amorphous aluminosilicate (SiO₂/Al₂O₃ ratio: 50) and that the molar formulation was changed as follows, a source composition having the following molar formulation was obtained by the same method as in Synthesis Example 1.
SiO₂/Al₂O₃ ratio = 83
NBA/SiO₂ ratio = 0.10
Na/SiO₂ ratio = 0.10
OH⁻/SiO₂ ratio = 0.10
H₂O/SiO₂ ratio = 11

Seed crystals (MFI zeolite; SiO₂/Al₂O₃ molar ratio, 2400) were mixed into 55 g of the source composition such that the seed crystal content was 1% by mass relative to 100% by mass of the source composition, and the resulting mixture was packed in a hermetically sealed container having a capacity of 80 mL. After packing, the composition was allowed to react by hydrothermal treatment at 150°C for 36 hours and under autogenous pressure while the container was rotated at 55 rpm, whereby a crystallized material was obtained. The resulting crystallized material was collected after solid-liquid separation, washing with purified water and drying at 110°C in an air atmosphere. The crystallized material (Hereinafter also referred to as "the crystallized material of Synthesis Example 3.") consisted of a single phase of an MFI zeolite and was an MFI zeolite (crystalline aluminosilicate) having a SiO₂/Al₂O₃ molar ratio of 80.

Then the resulting crystallized material (MFI zeolite) of Synthesis Example 3 was fired, subjected to ion exchange, washed and dried under the same conditions as in the firing, ion exchange, washing and drying performed for the crystallized material of Synthesis Example 1, whereby an MFI zeolite of this synthesis example was obtained.

The MFI zeolite of this synthesis example was a crystalline aluminosilicate having a SiO₂/Al₂O₃ molar ratio of 80 and a total pore volume of 0.21 cm³/g. The MFI zeolite of this synthesis example, furthermore, had an IR peak ratio of 0.1.

### SYNTHESIS EXAMPLE 4

Except that amorphous aluminosilicate (SiO₂/Al₂O₃ ratio: 216) was used instead of amorphous aluminosilicate (SiO₂/Al₂O₃ ratio: 50) and that the molar formulation was changed as follows, a source composition having the following molar formulation was obtained by the same method as in Synthesis Example 1.
SiO₂/Al₂O₃ ratio = 216
NBA/SiO₂ ratio = 0.10
Na/SiO₂ ratio = 0.10
OH⁻/SiO₂ ratio = 0.10
H₂O/SiO₂ ratio = 11

Seed crystals (MFI zeolite; SiO₂/Al₂O₃ molar ratio, 2400) were mixed into 55 g of the source composition such that the seed crystal content was 1% by mass relative to 100% by mass of the source composition, and the resulting mixture was packed in a hermetically sealed container having a capacity of 80 mL. After packing, the composition was allowed to react by hydrothermal treatment at 150°C for 36 hours and under autogenous pressure while the container was rotated at 55 rpm, whereby a crystallized material was obtained. The resulting crystallized material was collected after solid-liquid separation, washing with purified water and drying at 110°C in an air atmosphere. The crystallized material (Hereinafter also referred to as "the crystallized material of Synthesis Example 4.") consisted of a single phase of an MFI zeolite and was an MFI zeolite (crystalline aluminosilicate) having a SiO₂/Al₂O₃ molar ratio of 197.

Then the resulting crystallized material (MFI zeolite) of Synthesis Example 3 was fired, subjected to ion exchange, washed and dried under the same conditions as in the firing, ion exchange, washing and drying performed for the crystallized material of Synthesis Example 1, whereby an MFI zeolite of this synthesis example was obtained.

The MFI zeolite of this synthesis example was a crystalline aluminosilicate having a SiO₂/Al₂O₃ molar ratio of 206 and a total pore volume of 0.21 cm³/g. The MFI zeolite of this synthesis example, furthermore, had an IR peak ratio of 0.1.

### EXAMPLE 1

An aqueous solution of magnesium acetate was obtained by dissolving 5.29 g of magnesium acetate tetrahydrate in 4 g of purified water at 60°C. A 10.0-g portion of the MFI zeolite of Synthesis Example 1 and the aqueous solution of magnesium acetate were mixed in a mortar for 10 minutes at room temperature and atmospheric pressure. The resulting mixture was dried overnight at 110°C in an air atmosphere and then fired at 550°C for 2 hours in an air atmosphere, whereby an MFI zeolite of this example, supporting magnesium (hereinafter also referred to as "magnesium-containing MFI zeolite"), was obtained.

The magnesium-containing MFI zeolite of this example was an aluminosilicate having a SiO₂/Al₂O₃ ratio of 48 and a percentage by mass of element magnesium relative to the MFI zeolite of this example (hereinafter also referred to as "Mg content") of 6.0% by mass. The magnesium-containing MFI zeolite of this example, furthermore, had a total pore volume of 0.18 cm³/g and an IR peak ratio in the difference spectrum of 1.8.

### COMPARATIVE EXAMPLE 1

The MFI zeolite obtained in Synthesis Example 1 was used as the MFI zeolite of this comparative example.

### COMPARATIVE EXAMPLE 2

An aqueous solution of zinc nitrate was obtained by dissolving 0.49 g of zinc nitrate hexahydrate in 3.8 g of purified water. Except that this aqueous solution of zinc nitrate was used instead of the aqueous solution of magnesium acetate, an MFI zeolite of this comparative example, supporting zinc (hereinafter also referred to as "zinc-containing MFI zeolite"), was obtained by the same method as in Example 1.

The zinc-containing MFI zeolite of this comparative example was an aluminosilicate having a SiO₂/Al₂O₃ ratio of 48 and a percentage by mass of element zinc relative to the MFI zeolite of this comparative example (hereinafter also referred to as "Zn content") of 1.1% by mass. The zinc-containing MFI zeolite of this comparative example, furthermore, had a total pore volume of 0.25 cm³/g and an IR peak ratio in the difference spectrum of 0.0.

### EXAMPLE 2

Except that the zinc-containing MFI zeolite obtained in Comparative Example 2 was used instead of the MFI zeolite of Synthesis Example 1, an MFI zeolite of this example, supporting magnesium and zinc (hereinafter also referred to as "magnesium/zinc-containing MFI zeolite"), was obtained by the same method as in Example 1.

The magnesium/zinc-containing MFI zeolite of this example was an aluminosilicate having a SiO₂/Al₂O₃ ratio of 48, a Mg content of 6.0% by mass and a Zn content of 1.1% by mass. The magnesium/zinc-containing MFI zeolite of this example, furthermore, had a total pore volume of 0.19 cm³/g and an IR peak ratio in the difference spectrum of 1.7.

### EXAMPLE 3

An aqueous solution of calcium nitrate was obtained by dissolving 3.54 g of calcium nitrate tetrahydrate in 3 g of purified water. Except that the zinc-containing MFI zeolite obtained in Comparative Example 2 was used instead of the MFI zeolite of Synthesis Example 1 and that an aqueous solution of calcium nitrate was used instead of an aqueous solution of magnesium acetate, an MFI zeolite of this example, supporting calcium and zinc (hereinafter also referred to as "calcium/zinc-containing MFI zeolite"), was obtained by the same method as in Example 1.

The calcium/zinc-containing MFI zeolite of this example was an aluminosilicate having a SiO₂/Al₂O₃ ratio of 48, a percentage by mass of calcium relative to the MFI zeolite of this example (hereinafter also referred to as "Ca content") of 6.0% by mass and a Zn content of 1.1% by mass. The calcium/zinc-containing MFI zeolite of this example, furthermore, had a total pore volume of 0.20 cm³/g and an IR peak ratio in the difference spectrum of 3.6.

### EXAMPLE 4

An aqueous solution of diammonium hydrogen phosphate was obtained by dissolving 2.56 g of diammonium hydrogen phosphate in 4 g of purified water. Except that the zinc-containing MFI zeolite obtained in Comparative Example 2 was used instead of the MFI zeolite of Synthesis Example 1 and that an aqueous solution of diammonium hydrogen phosphate was used instead of an aqueous solution of magnesium acetate, an MFI zeolite of this example, supporting phosphorus and zinc (hereinafter also referred to as "phosphorus/zinc-containing MFI zeolite"), was obtained by the same method as in Example 1.

The phosphorus/zinc-containing MFI zeolite of this example was an aluminosilicate having a SiO₂/Al₂O₃ ratio of 48, a percentage by mass of element phosphorus relative to the MFI zeolite of this example (hereinafter also referred to as "P content") of 6.0% by mass and a Zn content of 1.1% by mass. The phosphorus/zinc-containing MFI zeolite of this example, furthermore, had a total pore volume of 0.14 cm³/g.

### EXAMPLE 5

An aqueous solution of zinc nitrate was obtained by dissolving 0.88 g of gallium nitrate n-hydrate (gallium nitrate content: 64.5% by mass) in 2.3 g of purified water. Except that this aqueous solution of gallium nitrate was used instead of the aqueous solution of zinc nitrate, an MFI zeolite of this example, supporting gallium (hereinafter also referred to as "gallium-containing MFI zeolite"), was obtained by the same method as in Comparative Example 2.

Except that the resulting gallium-containing MFI zeolite was used instead of the MFI zeolite of Synthesis Example 1, an MFI zeolite of this example, supporting magnesium and gallium (hereinafter also referred to as "magnesium/gallium-containing MFI zeolite"), was obtained by the same method as in Example 1.

The magnesium/gallium-containing MFI zeolite of this example was an aluminosilicate having a SiO₂/Al₂O₃ ratio of 48, a Mg content of 6.0% by mass and a percentage by mass of element gallium relative to the MFI zeolite of this example (hereinafter also referred to as "Ga content") of 1.7% by mass. The magnesium/gallium-containing MFI zeolite of this example, furthermore, had a total pore volume of 0.21 cm³/g and an IR peak ratio in the difference spectrum of 2.0.

### EXAMPLE 6

Except that the MFI zeolite of Synthesis Example 3 was used instead of the MFI zeolite of Synthesis Example 1 and that the amount of zinc nitrate hexahydrate added was changed from 0.49 g to 0.28 g, a zinc-containing MFI zeolite was obtained by the same method as in Comparative Example 2. Except that the resulting zinc-containing MFI zeolite was used instead of the MFI zeolite of Synthesis Example 1 and that the amount of magnesium acetate tetrahydrate added was changed from 5.29 g to 3.53 g, a magnesium/zinc-containing MFI zeolite of this example, supporting magnesium and zinc, was obtained by the same method as in Example 1.

The magnesium/zinc-containing MFI zeolite of this example was an aluminosilicate having a SiO₂/Al₂O₃ ratio of 80, a Mg content of 4.0% by mass and a Zn content of 0.66% by mass. The magnesium/zinc-containing MFI zeolite of this example, furthermore, had a total pore volume of 0.17 cm³/g and an IR peak ratio in the difference spectrum of 3.5.

### EXAMPLE 7

Except that the amount of magnesium acetate tetrahydrate added was changed from 3.53 g to 5.29 g, a magnesium/zinc-containing MFI zeolite of this example, supporting magnesium and zinc, was obtained by the same method as in Example 6.

The magnesium/zinc-containing MFI zeolite of this example was an aluminosilicate having a SiO₂/Al₂O₃ ratio of 80, a Mg content of 6.0% by mass and a Zn content of 0.66% by mass. The magnesium/zinc-containing MFI zeolite of this example, furthermore, had a total pore volume of 0.15 cm³/g and an IR peak ratio in the difference spectrum of 2.2.

### EXAMPLE 8

Except that the amount of magnesium acetate tetrahydrate added was changed from 3.53 g to 5.29 g and that the amount of zinc nitrate hexahydrate added was changed from 0.28 g to 0.34 g, a magnesium/zinc-containing MFI zeolite of this example, supporting magnesium and zinc, was obtained by the same method as in Example 6.

The magnesium/zinc-containing MFI zeolite of this example was an aluminosilicate having a SiO₂/Al₂O₃ ratio of 80, a Mg content of 6.0% by mass and a Zn content of 0.79% by mass. The magnesium/zinc-containing MFI zeolite of this example, furthermore, had a total pore volume of 0.13 cm³/g and an IR peak ratio in the difference spectrum of 2.1.

### EXAMPLE 9

Except that the amount of magnesium acetate tetrahydrate added was changed from 3.53 g to 5.29 g and that the amount of zinc nitrate hexahydrate added was changed from 0.28 g to 0.87 g, a magnesium/zinc-containing MFI zeolite of this example, supporting magnesium and zinc, was obtained by the same method as in Example 6.

The magnesium/zinc-containing MFI zeolite of this example was an aluminosilicate having a SiO₂/Al₂O₃ ratio of 80, a Mg content of 6.0% by mass and a Zn content of 2.0% by mass. The magnesium/zinc-containing MFI zeolite of this example, furthermore, had a total pore volume of 0.11 cm³/g and an IR peak ratio in the difference spectrum of 4.0.

### EXAMPLE 10

Except that the MFI zeolite of Synthesis Example 4 was used instead of the MFI zeolite of Synthesis Example 1 and that the amount of zinc nitrate hexahydrate added was changed from 0.49 g to 0.28 g, a zinc-containing MFI zeolite was obtained by the same method as in Comparative Example 2. Except that the resulting zinc-containing MFI zeolite was used instead of the MFI zeolite of Synthesis Example 1 and that the amount of magnesium acetate tetrahydrate added was changed from 5.29 g to 3.53 g, a magnesium/zinc-containing MFI zeolite of this example, supporting magnesium and zinc, was obtained by the same method as in Example 1.

The magnesium/zinc-containing MFI zeolite of this example was an aluminosilicate having a SiO₂/Al₂O₃ ratio of 206, a Mg content of 4.0% by mass and a Zn content of 0.66% by mass. The magnesium/zinc-containing MFI zeolite of this example, furthermore, had a total pore volume of 0.17 cm³/g and an IR peak ratio in the difference spectrum of 1.7.

### EXAMPLE 11

Except that a commercially available MEL zeolite (ZSM-11, manufactured by ACS Material LLC; SiO₂/Al₂O₃ ratio, 50) was used instead of the MFI zeolite of Synthesis Example 1, a zinc-containing MFI zeolite was obtained by the same method as in Comparative Example 2. Except that the resulting zinc-containing MFI zeolite was used instead of the MFI zeolite of Synthesis Example 1, a magnesium/zinc-containing MEL zeolite of this example, supporting magnesium and zinc, was obtained by the same method as in Example 1.

The magnesium/zinc-containing MEL zeolite of this example was an aluminosilicate having a SiO₂/Al₂O₃ ratio of 50, a Mg content of 6.0% by mass and a Zn content of 1.1% by mass. The magnesium/zinc-containing MEL zeolite of this example, furthermore, had a total pore volume of 0.17 cm³/g and an IR peak ratio in the difference spectrum of 3.2.

### EXAMPLE 12

An aqueous solution of calcium nitrate was obtained by dissolving 3.54 g of calcium nitrate tetrahydrate in 3 g of purified water. Except that an aqueous solution of calcium nitrate was used instead of an aqueous solution of magnesium acetate, an MFI zeolite of this example, supporting calcium (hereinafter also referred to as "calcium-containing MFI zeolite"), was obtained by the same method as in Example 1.

The calcium-containing MFI zeolite of this example was an aluminosilicate having a SiO₂/Al₂O₃ ratio of 48 and a Ca content of 6.0% by mass. The calcium-containing MFI zeolite of this example, furthermore, had a total pore volume of 0.21 cm³/g and an IR peak ratio in the difference spectrum of 5.0.

### COMPARATIVE EXAMPLE 3

Except that the crystallized material of Synthesis Example 1 was used instead of the MFI zeolite of Synthesis Example 1, an MFI zeolite of this comparative example, supporting magnesium (magnesium-containing MFI zeolite), was obtained by the same method as in Example 1.

The magnesium-containing MFI zeolite of this comparative example was an aluminosilicate having a SiO₂/Al₂O₃ ratio of 48 and a Mg content of 6.0% by mass. The magnesium-containing MFI zeolite of this comparative example, furthermore, had a total pore volume of 0.23 cm³/g and an IR peak ratio in the difference spectrum of 1.4.

### COMPARATIVE EXAMPLE 4

Except that the MFI zeolite of Synthesis Example 2 was used instead of the MFI zeolite of Synthesis Example 1, an MFI zeolite of this comparative example, supporting magnesium (magnesium-containing MFI zeolite), was obtained by the same method as in Example 1.

The magnesium-containing MFI zeolite of this comparative example was an aluminosilicate having a SiO₂/Al₂O₃ ratio of 48 and a Mg content of 6.0% by mass. The magnesium-containing MFI zeolite of this comparative example, furthermore, had a total pore volume of 0.62 cm³/g and an IR peak ratio in the difference spectrum of 1.5.

### COMPARATIVE EXAMPLE 5

A magnesium-containing MFI zeolite of this comparative example was prepared by the same method as in Comparative Example 4, except that the amount of magnesium acetate tetrahydrate added was changed from 5.29 g to 10.59 g.

The magnesium-containing MFI zeolite of this comparative example was an aluminosilicate having a SiO₂/Al₂O₃ ratio of 48 and a Mg content of 12.0% by mass. The magnesium-containing MFI zeolite of this comparative example, furthermore, had a total pore volume of 0.52 cm³/g.

### COMPARATIVE EXAMPLE 6

A gallium-containing MFI zeolite of this comparative example was obtained by the same method as in Example 5. That is, an aqueous solution of zinc nitrate was obtained by dissolving 0.88 g of gallium nitrate n-hydrate (gallium nitrate content: 64.5% by mass) in 2.3 g of purified water. Except that this aqueous solution of gallium nitrate was used instead of the aqueous solution of zinc nitrate, a gallium-containing MFI zeolite of this comparative example was obtained by the same method as in Comparative Example 2.

The gallium-containing MFI zeolite of this comparative example was an aluminosilicate having a SiO₂/Al₂O₃ ratio of 48 and a Ga content of 1.7% by mass. The gallium-containing MFI zeolite of this comparative example, furthermore, had a total pore volume of 0.24 cm³/g.

### COMPARATIVE EXAMPLE 7

A magnesium/zinc-containing MFI zeolite of this comparative example was prepared by the same method as in Example 6, except that the amount of magnesium acetate tetrahydrate added was changed from 3.53 g to 1.76 g.

The magnesium/zinc-containing MFI zeolite of this comparative example was an aluminosilicate having a SiO₂/Al₂O₃ ratio of 80, a Mg content of 2.0% by mass and a Zn content of 0.66% by mass. The magnesium/zinc-containing MFI zeolite of this comparative example, furthermore, had a total pore volume of 0.22 cm³/g.

### COMPARATIVE EXAMPLE 8

Except that the amount of zinc nitrate hexahydrate added was changed from 0.49 g to 0.28 g, a zinc-containing MFI zeolite was obtained by the same method as in Comparative Example 2. Except that the resulting zinc-containing MFI zeolite was used instead of the MFI zeolite of Synthesis Example 1 and that the amount of magnesium acetate tetrahydrate added was changed from 5.29 g to 3.53 g, a magnesium/zinc-containing MFI zeolite of this comparative example was prepared by the same method as in Example 1.

The magnesium/zinc-containing MFI zeolite of this comparative example was an aluminosilicate having a SiO₂/Al₂O₃ ratio of 48, a Mg content of 4.0% by mass and a Zn content of 0.66% by mass. The magnesium/zinc-containing MFI zeolite of this comparative example, furthermore, had a total pore volume of 0.24 cm³/g and an IR peak ratio in the difference spectrum of 1.2.

### COMPARATIVE EXAMPLE 9

An aqueous solution of zinc nitrate was obtained by dissolving 0.49 g of zinc nitrate hexahydrate in 3.8 g of purified water. Except that the magnesium-containing MFI zeolite of Example 1 was used instead of the MFI zeolite of Synthesis Example 1 and that the above aqueous solution of zinc nitrate was used instead of the aqueous solution of magnesium acetate, an MFI zeolite of this comparative example, supporting zinc and magnesium (hereinafter also referred to as "zinc/magnesium-containing MFI zeolite"), was obtained by the same method as in Example 1.

The zinc/magnesium-containing MFI zeolite of this comparative example was an aluminosilicate having a SiO₂/Al₂O₃ ratio of 48, a Zn content of 1.1% by mass and a Mg content of 6.0% by mass. The zinc/magnesium-containing MFI zeolite of this comparative example, furthermore, had a total pore volume of 0.22 cm³/g.

### COMPARATIVE EXAMPLE 10

An aqueous solution of nickel nitrate was obtained by dissolving 0.43 g of nickel(II) nitrate hexahydrate in 2 g of purified water. Except that an aqueous solution of nickel nitrate was used instead of an aqueous solution of magnesium acetate, an MFI zeolite of this comparative example, supporting nickel (hereinafter also referred to as "nickel-containing MFI zeolite"), was obtained by the same method as in Example 1.

The nickel-containing MFI zeolite of this comparative example was an aluminosilicate having a SiO₂/Al₂O₃ ratio of 48 and a percentage by mass of element nickel relative to the MFI zeolite of this comparative example (hereinafter also referred to as "Ni content") of 1.0% by mass. The nickel-containing MFI zeolite of this comparative example, furthermore, had a total pore volume of 0.17 cm³/g.

### COMPARATIVE EXAMPLE 11

A 7.06-g portion of an aqueous solution of No. 3 sodium silicate (containing 14.5% by mass Si as SiO₂ and 4.5% by mass Na as Na₂O) and 10.0 g of the MFI zeolite of Synthesis Example 1 were mixed in a mortar for 10 minutes. The resulting mixture was dried overnight at 110°C in an air atmosphere and then fired at 550°C for 2 hours in an air atmosphere, whereby an MFI zeolite of this comparative example, supporting silica (hereinafter also referred to as "silica-supporting MFI zeolite"), was obtained.

The silica-supporting MFI zeolite of this comparative example had a SiO₂/Al₂O₃ ratio of 58 and a percentage by mass of supported element silicon relative to the MFI zeolite of this comparative example relative to the MFI zeolite of this comparative example (hereinafter also referred to as "Si content") of 8.4% by mass. The silica-supporting MFI zeolite of this comparative example, furthermore, had a total pore volume of 0.13 cm³/g and an IR peak ratio in the difference spectrum of 0.0.

Characteristics of the zeolites of the examples and comparative examples are presented in Tables 1 and 2 below. It should be noted that in Tables 1 and 2 below, SAR represents the SiO₂/Al₂O₃ ratio.

**[Table 1]**

| | Specified element | | Additional element | | Total pore volume [cm³/g] | SAR [-] | IR peak ratio [-] |
|---|---|---|---|---|---|---|---|
| | Type | Amount [wt%] | Type | Amount [wt%] | | | |
| Example 1 | Mg | 6.0 | - | - | 0.18 | 48 | 1.8 |
| Example 2 | Mg | 6.0 | Zn | 1.1 | 0.19 | 48 | 1.7 |
| Example 3 | Ca | 6.0 | Zn | 1.1 | 0.20 | 48 | 3.6 |
| Example 4 | P | 6.0 | Zn | 1.1 | 0.14 | 48 | - |
| Example 5 | Mg | 6.0 | Ga | 1.7 | 0.21 | 48 | 2.0 |
| Example 6 | Mg | 4.0 | Zn | 0.66 | 0.17 | 80 | 3.5 |
| Example 7 | Mg | 6.0 | Zn | 0.66 | 0.15 | 80 | 2.2 |
| Example 8 | Mg | 6.0 | Zn | 0.79 | 0.13 | 80 | 2.1 |
| Example 9 | Mg | 6.0 | Zn | 2.0 | 0.11 | 80 | 4.0 |
| Example 10 | Mg | 4.0 | Zn | 0.66 | 0.17 | 206 | 1.7 |
| Example 11 | Mg | 6.0 | Zn | 1.1 | 0.17 | 50 | 3.2 |
| Example 12 | Ca | 6.0 | - | - | 0.21 | 48 | 5.0 |

**[Table 2]**

| | Specified element | | Additional element | | Total pore volume [cm³/g] | SAR [-] | IR peak ratio [-] |
|---|---|---|---|---|---|---|---|
| | Type | Amount [wt%] | Type | Amount [wt%] | | | |
| Comparative Example 1 | - | - | - | - | 0.25 | 48 | 0.1 |
| Comparative Example 2 | - | - | Zn | 1.1 | 0.25 | 48 | 0.0 |
| Comparative Example 3 | Mg | 6.0 | - | - | 0.23 | 48 | 1.4 |
| Comparative Example 4 | Mg | 6.0 | - | - | 0.62 | 48 | 1.5 |
| Comparative Example 5 | Mg | 12.0 | - | - | 0.52 | 48 | - |
| Comparative Example 6 | - | - | Ga | 1.7 | 0.24 | 48 | - |
| Comparative Example 7 | Mg | 2.0 | Zn | 0.66 | 0.22 | 80 | - |
| Comparative Example 8 | Mg | 4.0 | Zn | 0.66 | 0.24 | 48 | 1.2 |
| Comparative Example 9 | Mg | 6.0 | Zn | 1.1 | 0.22 | 48 | - |
| Comparative Example 10 | - | - | Ni | 1.0 | 0.17 | 48 | - |
| Comparative Example 11 | - | - | Si | 8.4 | 0.13 | 58 | 0.0 |

As can be understood from the results for Comparative Examples 1, 2 and 6 presented in the above tables, simply supporting an additional element, such as zinc or gallium, on an oxygen ten-membered ring zeolite did not result in a significant change in total pore volume. As is clear from comparison between Comparative Example 2 and Examples 2, 3 and 4 and comparison between Comparative Example 6 and Example 5, however, supporting a specified element, such as magnesium, calcium or phosphorus, on an oxygen ten-membered ring zeolite resulted in a decrease in total pore volume. From these results, it was presumed that for the oxygen ten-membered ring zeolites of Examples 1 to 12, at least part of the specified element, such as magnesium, calcium or phosphorus, was supported on the internal surfaces of pores in the zeolite, resulting in a decrease in total pore volume. It was also understood that in Comparative Example 3, in which magnesium was supported on an oxygen ten-membered ring zeolite that was not subjected to firing treatment (zeolite without SDA removal treatment), and in Comparative Example 7, in which the Mg content was lower compared with those in the other examples and comparative examples, the total pore volume was less likely to decrease, and magnesium was less likely to be supported on the internal surfaces of pores.

As can be understood from the IR peak ratios in Example 1 and Comparative Example 1 presented in the above tables, furthermore, the IR peak ratio increased as a result of supporting magnesium on an MFI zeolite. Moreover, as can be understood from the IR peak ratios in Examples 2 to 4 and Comparative Example 2 presented in Table 1 above, the IR peak ratio increased as a result of supporting a specified element, such as calcium, magnesium or phosphorus, on a zinc-containing MFI zeolite. From these results, it was understood that the specified elements, such as magnesium, calcium and phosphorus, are likely to be supported on the external surface of a zeolite. As can be understood from the IR peak ratios in Comparative Example 1 and Comparative Example 2, however, supporting zinc on an MFI zeolite resulted in little change in the IR peak ratio. From this result, it was presumed that for the MFI zeolite of Comparative Example 2, zinc was supported at a site other than the external surface of the MFI zeolite (e.g., inside the MFI zeolite).

### MEASUREMENT EXAMPLE 1

### <Methanol Reforming Reaction>

Using a fixed-bed flow reactor, 2 g of a measurement sample was packed into a stainless-steel reaction column having an inner diameter of 8 mm. Through this reaction column, nitrogen was passed for 1 hour at a flow rate of 50 mL/min, a temperature of 450°C and 0.2 MPa (gauge pressure) as a pretreatment (activation treatment). After the pretreatment, nitrogen supplied at a flow rate of 20 mL/min and gaseous methanol obtained by vaporizing, at 65°C, liquid methanol supplied at 2 g/min were supplied to the reaction column, whereby a gas mixture of nitrogen and methanol (nitrogen/methanol mixed gas) was passed through the reaction column such that the conditions were 420°C, 0.2 MPa (gauge pressure) and a weight hourly space velocity of methanol of 1.0 Hr⁻¹. Six hours after the start of passing the mixed gas, the methanol concentrations at the inlet and outlet of the fixed-bed flow reaction column were measured. In addition, analysis of the product was performed using gas chromatography.

From the results obtained, methanol conversion (%), p-xylene selectivity (%) and the BTX yield were determined using formulae (2), (3) and (4), respectively, below.$\begin{matrix}Methanol conversion \left(%\right) \\ = \left\{\left(\left[methanol\right]in-\left[methanol\right]out\right)/\left[methanol\right]in\right\} \times \\ 100\end{matrix}$

In formula (2) above, [methanol]in is the methanol concentration (v/v%) at the inlet of the fixed-bed flow reaction column, and [methanol]out is the methanol concentration (v/v%) at the outlet of the fixed-bed flow reaction column.$\begin{matrix}p - xylene selectivity \left(%\right) \\ = \left(\left[p-xylene\right]out/\left[xylene\right]out\right) \times 100\end{matrix}$

In formula (3) above, [p-xylene]out is the total number of moles (mol) of carbon atoms contained in p-xylene at the outlet of the fixed-bed flow reaction column, and [xylene]out is the total number of moles (mol) of carbon atoms contained in xylene (p-xylene, o-xylene and m-xylene) at the outlet of the fixed-bed flow reaction column. $\begin{matrix}BTX yield \left(%\right) = \\ \left(\left[BTX\right]out/\left[product\right]out\right) \times methanol conversion\end{matrix}$

In formula (4) above, [BTX]out is the total number of moles (mol) of carbon atoms contained in benzene, xylene (p-xylene, o-xylene and m-xylene) and toluene at the outlet of the fixed-bed flow reaction column, [product]out is the total number of moles (mol) of carbon atoms contained in the product (all components in the product) at the outlet of the fixed-bed flow reaction column, and the methanol conversion is the methanol conversion (%) determined according to formula (2) above.

**[Table 3]**

| | Methanol conversion | p-xylene selectivity | BTX yield |
|---|---|---|---|
| | [%] | [%] | [%] |
| Example 1 | 100 | 73 | 27 |
| Example 2 | 100 | 63 | 46 |
| Example 3 | 100 | 43 | 16 |
| Example 4 | 100 | 49 | 3 |
| Example 5 | 100 | 62 | 29 |
| Example 6 | 100 | 92 | 34 |
| Example 7 | 100 | 91 | 28 |
| Example 8 | 100 | 82 | 31 |
| Example 9 | 100 | 87 | 28 |
| Example 10 | 100 | 68 | 30 |
| Example 11 | 100 | 85 | 32 |
| Example 12 | 100 | 47 | 12 |

**[Table 4]**

| | Methanol conversion | p-xylene selectivity | BTX yield |
|---|---|---|---|
| | [%] | [%] | [%] |
| Comparative Example 1 | 100 | 24 | 31 |
| Comparative Example 2 | 100 | 24 | - |
| Comparative Example 3 | 100 | 26 | 26 |
| Comparative Example 4 | 100 | 24 | 23 |
| Comparative Example 5 | 100 | 24 | - |
| Comparative Example 6 | 100 | 24 | - |
| Comparative Example 7 | 100 | 25 | - |
| Comparative Example 8 | 100 | 25 | - |
| Comparative Example 9 | 100 | 27 | - |
| Comparative Example 10 | 100 | 24 | - |
| Comparative Example 11 | 100 | 32 | - |

As shown in Tables 3 and 4 above, with the zeolites of the examples, compared with the zeolites of the comparative examples, p-xylene selectivity was successfully increased in the manufacture of xylene by alcohol reforming.

## Claims

1. A zeolite having an oxygen ten-membered ring structure, the zeolite comprising one or more specified elements selected from the group of magnesium, calcium, titanium, boron and phosphorus, wherein a total pore volume is 0.21 cm³/g or less.

2. The zeolite according to claim 1, wherein the zeolite having an oxygen ten-membered ring structure is an MFI zeolite, an MEL zeolite, a TON zeolite, an STF zeolite, an MTT zeolite, an MWW zeolite or a ZSM-48 zeolite.

3. The zeolite according to claim 1 or 2, wherein a molar ratio of silica to alumina is 10 or greater and 250 or less.

4. The zeolite according to any one of claims 1 to 3, further comprising one or more additional elements selected from the group of silver, zinc, gallium and iron.

5. The zeolite according to any one of claims 1 to 4, wherein the specified elements are supported at least on an external surface.

6. The zeolite according to any one of claims 1 to 5, wherein in a difference spectrum obtained by subtracting an IR spectrum of the zeolite before adsorption of 2,6-di-tert-butylpyridine from an IR spectrum of the zeolite with 2,6-di-tert-butylpyridine adsorbed thereonto, a ratio of a maximum intensity of a peak having a peak top within a range of 1630 cm⁻¹ to 1650 cm⁻¹, inclusive, to a maximum intensity of a peak having a peak top within a range of 1600 cm⁻¹ to 1620 cm⁻¹, inclusive, is greater than 1.5.

7. The zeolite according to any one of claims 1 to 6, wherein when the zeolite is subjected to a treatment of bringing a gas mixture of nitrogen and methanol into contact therewith under conditions of a reaction temperature of 420°C, a reaction pressure (gauge pressure) of 0.2 MPa, a duration of reaction of 6 hours and a methanol weight hourly space velocity of 1.0 Hr⁻¹, p-xylene selectivity is 40% or more.

8. A catalyst for alcohol reforming, the catalyst comprising the zeolite according to any one of claims 1 to 7.

9. A method for manufacturing xylene, the method comprising bringing the zeolite according to any one of claims 1 to 7 and a fluid containing an alcohol into contact.
